# EUROPÄISCHE PATENTSCHRIFT

(11) **EP 1 742 583 B1**
(45) Veröffentlichungstag und Bekanntmachung des Hinweises auf die Patenterteilung: **13.03.2013**
(21) Anmeldenummer: 05735803.8
(22) Anmeldetag: 20.04.2005
(51) Int. Cl.: A61B 17/02, A61B 19/00

(54) **EINRICHTUNG ZUM ERFASSEN EINER KRAFT-WEG-KENNLINIE EINES ODER MEHRERER BÄNDER**
DEVICE FOR ACQUIRING A FORCE-DISTANCE CHARACTERISTIC OF ONE OR MORE LIGAMENTS
DISPOSITIF PERMETTANT DE DETECTER UNE COURBE CARACTERISTIQUE FORCE-TRAJET D'AU MOINS UN LIGAMENT

(30) Priorität: 22.04.2004 DE 102004019655; 25.05.2004 DE 102004025612
(43) Veröffentlichungstag der Anmeldung: 17.01.2007
(73) Patentinhaber: Smith & Nephew Orthopaedics AG, 6343 Rotkreuz (CH)
(72) Erfinder: MOSER, Walter, CH-3126 Kaufdorf (CH); RITSCHL, Peter, A-1140 Wien (AT); BROERS, Holger, 26670 Uplengen-Spols (DE)
(74) Vertreter: Popp, Eugen
(86) Internationale Anmeldenummer: PCT/EP2005/004241
(87) Internationale Veröffentlichungsnummer: WO 2005/102178

(56) Entgegenhaltungen:
- DE-U1- 20 217 014
- US-A- 4 899 761
- US-A1- 2002 156 480
- US-A1- 2003 036 764
- US-A1- 2005 038 442

## Beschreibung

Die vorliegende Erfindung betrifft eine Vorrichtung zum Spannen von Bändern, sowie eine Einrichtung zum Erfassen eines Kraft-Weg-Verhältnisses oder einer Kraft-Weg-Kennlinie eines oder mehrerer Bänder, wobei die vorgenannte Vorrichtung ein wesentlicher Bestandteil derselben ist, gemäß den Oberbegriffen der Ansprüche 1 und 8.

Vorrichtungen zum Spannen von Bändern, insbesondere Gelenkbändern eines menschlichen oder tierischen Körpers dienen einer kontrollierten Anspannung von Weichteilstrukturen bei einer Operation zum Ersatz von erkrankten Gelenken durch Kunstgelenke. Bekannte Beispiele hierfür sind der Ersatz von Hüftgelenken, Kniegelenken sowie der Ersatz von Wirbelgelenken durch künstliche Gelenke.

Wichtig für den Erfolg einer Operation zum Ersatz eines beschädigten Gelenkes durch ein künstliches Gelenk ist eine korrekte Einstellung der das Kunstgelenk stabilisierenden passiven Strukturen, d.h. der Bänder und der das Gelenk umgebenden Kapsel.

In der neueren Literatur zum Thema Gelenkersatz wird die Bedeutung der quantitativen Beurteilung der Kapsel-Bandstrukturen für den Operationserfolg beschrieben (vgl. z.B. "An In-Vivo Biomechanical Analysis of the Soft-Tissue Envelope of Osteoarthritic Knees" in "The Journal of Arthroplasty", Vol. 19, 2004). Die in dieser Schrift zusammengefaßten Erkenntnisse führen zu einer Operationstechnik, welche generell von einer konstanten Steifigkeit der Bänder ausgeht.

Allerdings wurden bei den dort angestellten Untersuchungen erhebliche Streuungen der Steifigkeiten des Kapsel-Band-Apparates von bis zu 30% registriert. Die mechanischen Eigenschaften des Kapsel-Band-Apparates sind demnach individuell sehr unterschiedlich. Zudem sind diese abhängig vom Krankheitszustand des Gelenkes und vom Endzustand des Kapsel-Band-Apparates, beim Knie insbesondere vom Zustand des hinteren Kreuzbandes. Somit lassen sich nur näherungsweise Angaben für die zu verwendenden Spreizkräfte machen. Unterschiede ergeben sich u.a. durch das zu bewegende unterschiedliche Beingewicht, das Alter des Patienten und die Art der Erkrankung. Besonders kräftige Strukturen sind bei relativ jungen Patienten mit posttraumatischer Arthrose zu erwarten. Bei Altersarthrosen und beim rheumatischen Erkrankungen ist mit sinkender Qualität der Strukturen zu rechnen.

Wird bei bzw. nach der Operation keine Stabilität des Gelenks in Beugung erreicht, resultiert daraus eine nicht-optimale Kinematik des Kunstgelenks. Ein bekanntes Beispiel hierfür ist beim Ersatz des Kniegelenks durch ein künstliches Gelenk das Vorwärtsgleiten einer Oberschenkelkomponente auf einer Unterschenkelkomponente mit zunehmender Kniebeugung, was exakt im Gegensatz zum natürlichen Bewegungsablauf des Kniegelenks steht.

Eine optimale Gelenkkinematik ist bei nahezu isometrisch beanspruchten Bandstrukturen zu erwarten. Operationstechnisch wird dies durch Einstellung der Gelenkstabilität in zwei Positionen erreicht. Bei Ersatz eines Kniegelenks wird die Beurteilung in 90° Beugung und in Streckung vorgenommen. Eine optimale Stabilität des Gelenks kann in einer Kraft-Weg-Kennlinie leicht aufgefunden werden. An diesem "Stabilitätspunkt" sind alle Strukturen gerade leicht angespannt, jedoch noch nicht elastisch gedehnt. Wie bereits vorstehend erwähnt, ist dieser Stabilitätspunkt patientenspezifisch. Es ergibt sich also die Notwendigkeit, eine Kraft-Weg-Kennlinie des zu operierenden Gelenkes aufzuzeichnen, um so die notwendige Information zur Festlegung des Stabilitätspunktes zu erhalten. Eine derartige Aufzeichnung ist bis heute wegen der Anforderungen an ein Operationsinstrument nicht realisiert worden.

Gemäß dem Stand der Technik kommen relativ einfach konstruierte Vorrichtungen zum Spannen von Bändern zum Einsatz, die teilweise auch mit einer mechanischen Kraftanzeige zur Anzeige der Kraft, mit der ein Gelenk gespreizt wird, und einer Anzeige des Spreizweges, d.h. also des Weges, um den zwei Gelenkkompartimente gespreizt werden, versehen sind. Eine derartige Vorrichtung zum Spannen von Bändern ist beispielsweise in der WO 00/78225 A1 dargestellt.

US-A-2003/0036764 offenbart ebenfalls eine Vorrichtung zum Spannen von Bändern mit Anzeige von Kraft und Spreizweg (Fig. 73).

US-A-2003/0036

Eine weitere Vorrichtung zum Spreizen von Bändern geht auf die Anmelderin zurück. Die besagte Vorrichtung besteht aus zwei Spreizelementen, die mittels zweier voneinander unabhängiger Handhaben über eine Schraubendruckfeder betätigbar sind. Das Ausrücken der Spreizelemente erfolgt durch eine Kraft, die durch ein Zusammendrücken der Schraubendruckfeder mittels der jeweiligen Handhabe erzeugt wird. An der Vorrichtung kann neben der jeweils auf das betreffende Spreizelement ausgeübten Kraft auch der von diesem jeweils zurückgelegte Weg abgelesen werden, so daß ein entsprechendes Kraft-Weg-Verhältnis gebildet werden kann. Die Anzeige sowohl der Kraft, als auch des vom Spreizelement zurückgelegten Weges erfolgt auf einer Skala, die an der Vorrichtung angebracht ist und wird vom Operateur abgelesen.

Mit den vorstehend erwähnten Vorrichtungen zum Spannen von Bändern, insbesondere von Gelenkbändern, ist es nicht möglich, eine präzise Kraft-Weg-Kennlinie mit einer hohen Punktdichte aufzunehmen. Neben Ableseungenauigkeiten, die durch eine Ablesung der Meßwerte durch den Operateur bzw. Hilfspersonal bedingt sind, ist die für eine exakte Kennlinie nötige Punktdichte nicht ermittelbar, da dies die Operationsdauer unverhältnismäßig verlängern würde.

Der Erfindung liegt demnach die Aufgabe zugrunde, eine Vorrichtung zum Spannen von Bändern anzugeben, mit deren Hilfe im Rahmen einer ebenfalls anzugebenden Einrichtung zum Erfassen einer Kraft-Weg-Kennlinie, eine Kraft-Weg-Kennlinie mit möglichst hoher Präzision ermittelt werden kann, um somit einen Stabilitätspunkt eines Gelenkes optimal festlegen zu können. Ferner ist es Aufgabe der Erfindung, ein entsprechendes Verfahren zur Ermittlung einer Kraft-Weg-Kennlinie anzugeben.

Diese Aufgabe wird in ihrem ersten Aspekt durch eine Vorrichtung zum Spannen von Bändern mit den Merkmalen des Anspruchs 1 und hinsichtlich der Einrichtung zum Erfassen einer Kraft-Weg-Kennlinie durch eine Einrichtung mit den Merkmalen des Patentanspruchs 8 gelöst.

Die erfindungsgemäße Vorrichtung zum Spannen von Bändern, insbesondere Gelenkbändern eines menschlichen oder eines tierischen Körpers umfaßt mindestens eine Handhabe, wobei die eine Handhabe bzw. bei Vorliegen mehrerer Handhaben jede Handhabe mit jeweils einem der betreffenden Handhabe zugeordneten Spannelement in Wirkeingriff steht. Ferner ist der einen oder jeder der mehreren Handhaben jeweils eine Kraftanzeige zugeordnet, welche eine auf ein der Handhabe zugeordnetes Spannelement ausgeübte Kraft anzeigt, wenn ein oder mehrere Bänder gespannt werden. Ferner ist jedem Spannelement eine Weganzeige zugeordnet, die die Auslenkung des jeweiligen Spannelements aus einer Referenzposition anzeigt. Der einen oder jeder der mehreren Kraftanzeigen und der einen bzw. jeder der mehreren Weganzeigen ist jeweils mindestens ein Geber zugeordnet, welcher durch ein der Vorrichtung zugeordnetes Meßwert-Erfassungssystem berührungslos erfaßbar ist. Selbstverständlich können den beiden vorgenannten Anzeigen jeweils auch mehrere Geber zugeordnet sein. Die Vorrichtung zeichnet sich dadurch aus, dass wenigstens ein Teil der vorhandenen Geber retro-reflektierende Kugeln aufweist, welche insbesondere Teil eines optischen Meßsystems, z.B. eines chirurgischen Navigationssystems sind und die (als ein passives Bauelement) eine leichte Handhabbarkeit und ein geringes Gewicht der Vorrichtung ermöglichen. Durch diesen Aufbau wird es möglich, eine Kraft-Weg-Kennlinie eines oder mehrerer Bänder, insbesondere Gelenkbänder, aufzunehmen und somit den Stabilitätspunkt eines Gelenkes zu bestimmen. Ferner sorgt die berührungslose Erfassung der Meßdaten dafür, daß hohe Sterilitätsansprüche während der Operation gewahrt bleiben können.

In einer bevorzugten Ausführungsform weisen der oder die Geber optische Elemente auf. Zusätzlich können der oder die Geber magnetische und/oder elektromagnetische Elemente aufweisen. Sowohl optische Elemente, als auch magnetische und/oder elektromagnetische Elemente sind leicht und präzise durch zugeordnete Einrichtungen erfaßbar, wobei optische Elemente am einfachsten erfaßbar sind, jedoch auch ein freies Blickfeld auf die Apparatur benötigen; magnetische und/oder elektromagnetische Elemente sind etwas aufwendiger zu detektieren, jedoch kann das Blickfeld zwischen Geber und Apparatur optisch versperrt sein.

Das bzw. jedes Spannelement ist vorzugsweise mechanisch, elektromotorisch, hydraulisch, pneumatisch oder hydro-pneumatisch betätigbar. Eine derartige Bedienung ist zum Teil, insbesondere im mechanischen bzw. im elektromotorischen Fall konstruktiv relativ einfach zu bewerkstelligen, eine hydraulische, pneumatische oder hydropneumatische Bedienung bietet für den Operateur einen hohen Komfort, wobei keine (möglicherweise empfindliche, im OP in Verwendung befindliche, elektronische Geräte störenden) elektromagnetischen Störquellen vorhanden sind.

Die Betätigungseinrichtung für das Spannelement umfaßt in einer bevorzugten Ausführungsform ein krafterzeugendes Element, wobei die Messung der auf das jeweilige Spannelement wirkenden Kraft durch eine Messung einer dem jeweiligen krafterzeugenden Element zugeordneten Wegdifferenz bewerkstelligt wird. Zusätzlich oder aber auch alternativ kann auch die Auslenkung aus einer Referenzposition, die beispielsweise eine Null-Position oder dergleichen sein kann, gemessen werden. Diese Ausführungsform gewährleistet eine einfache Messung mit leicht zugänglichen Meßgrößen.

In einer konstruktiv besonders einfachen Ausführungsform ist das krafterzeugende Element eine Schraubendruckfeder. Der Vorteil ist eine günstige Herstellung, sowie eine relativ hohe Robustheit der erfindungsgemäßen Vorrichtung.

Um eine große Bewegungsfreiheit des Operateurs hinsichtlich der Position des zu ersetzenden Gelenks zu gewährleisten, ist an der Vorrichtung zur Spannung der Bänder vorzugsweise mindestens ein Geber für die Bestimmung einer Achsausrichtung der Vorrichtung angebracht. Der Patient kann demnach währen der Operation in eine für den Operateur (der jeweiligen Situation angepaßt) günstige Lage gebracht werden.

Der vorgenannte Geber bzw. die Geber für die Bestimmung der Achsausrichtung weisen vorzugsweise magnetische und/oder elektromagnetische und/oder optische Elemente auf, wobei die vorstehend erwähnten Geber bevorzugt demselben Wirkprinzip wie die weiteren in der Vorrichtung vorhandenen Geber unterliegen. Dies stellt eine einfache Messung mit einem minimalen apparativen Aufwand sicher.

Ein weiterer wesentlicher Punkt der Erfindung ist es, daß eine Einrichtung zum Erfassen eines Kraft-Weg-Verhältnisses oder einer Kraft-Weg-Kennlinie eines oder mehrerer tierischer oder menschlicher Bänder, insbesondere Gelenkbänder, eine Vorrichtung zum Spannen von Bändern nach einer oder mehreren Ausführungsformen der vorstehend beschriebenen Vorrichtung zum Spannen von Bändern aufweist, sowie Mittel zur Erfassung der Geber und Mittel zur Auswertung der erfaßten Daten. Eine derartige Einrichtung ermöglicht es, die Geber berührungslos zu erfassen und so eine Kraft-Weg-Kennlinie aufzustellen, wobei die jeweiligen Vorteile u.a. denen der zugehörigen Vorrichtung entsprechen.

In einer bevorzugten Ausführungsform weist die Einrichtung ferner einen Speicher und eine Recheneinheit auf. In diesen werden die erfaßten und/oder ausgewerteten Daten und/oder Referenzwerte gespeichert und ausgewertet. Alternativ oder zusätzlich können auch spezifische Daten hinsichtlich der Raumlage des zu vermessenden Band-Apparates im Speicher gehalten werden. Dadurch wird eine kontinuierliche Messung ermöglicht, wobei insbesondere bei der Speicherung von Daten hinsichtlich der Raumlage des zu vermessenden Band-Apparates Referenzdaten zur Verfügung gestellt werden können.

Vorzugsweise erfolgen die durchzuführenden Positionsbestimmungen in etwa zeitgleich, d.h. möglichst nur mit einer durch die verarbeitende Einheit bzw. die der Meßeinheit übergeordneten Einheit bedingten Verzögerung. Diese Verzögerung entsteht im allgemeinen beispielsweise dadurch, daß Befehle in einem Rechner nicht exakt parallel ausgeführt werden können, sondern sequenziell ausgeführt werden müssen. Ferner besteht die Möglichkeit von Totzeiten der jeweiligen Meßapparaturen. Die Taktfrequenzen sind (insbesondere bei Rechnern) jedoch so hoch, daß diese Zeitverzögerungen zwischen den Messungen üblicherweise sehr gering sind. Die vorstehend erwähnten Messungen sind die Messung der auf ein Spannelement wirkenden Kraft bzw. der auf die Spannelemente wirkenden Kräfte, eine Messung der dem Spannelement zugeordneten Wegdifferenz und/oder der Auslenkung aus einer Referenzposition bzw. von den Spannelementen zugeordneten Wegdifferenzen und/oder Auslenkungen derselben aus einer Referenzposition, sowie gegebenenfalls eine Messung der Achsausrichtung der Vorrichtung zum Spannen von Bändern. Der Vorteil der gleichzeitigen Messung besteht darin, daß die Meßgenauigkeit der Kraft-Weg-Kennlinie erhöht wird, da jedem entsprechenden Kraftwert ein simultan bzw. nahezu simultan gemessener Weg-Wert zugeordnet wird. Die gegebenenfalls gleichzeitig stattfindende Messung der Achsausrichtung der Vorrichtung zum Spannen von Bändern erhöht die Flexibilität des Operateurs, der besagte Vorrichtung nicht während der ganzen Operation in ein und derselben Achsausrichtung belassen muß.

Die Vorrichtung weist weiterhin in einer bevorzugten Ausführungsform Mittel zur Erfassung zeitabhängiger Änderungen verschiedener Meßgrößen auf. Insbesondere von Interesse sind die zeitabhängigen Änderungen von auf zugeordnete Spannelemente ausgeübten Kräften und/oder von krafterzeugenden Elementen zugeordneten Wegdifferenzen und/oder Auslenkungen aus einer Referenzposition. Durch die Messung der verschiedenen Größen in Abhängigkeit der Zeit können zeitabhängige Veränderungen im Band-Apparat des Patienten errechnet und sichtbar gemacht werden, womit sich viskoelastische Eigenschaften des biologischen Gewebes messen und beurteilen lassen. Selbstverständlich gehört zu den Größen, die über die Zeit betrachtet werden können und gegebenenfalls auch beobachtet werden müssen, auch die Achsausrichtung der Vorrichtung zum Spannen von Bändern.

Eine konstruktiv einfache bevorzugte Ausführungsform umfaßt beim Einsatz von optischen Gebern eine Stereo-Kamera zur Erfassung der jeweiligen Geber.

Ein Verfahren zur Erfassung eines Kraft-Weg-Verhältnisses bzw. einer Kraft-Weg-Kennlinie eines oder mehrerer tierischer oder menschlicher Bänder, insbesondere Gelenkbänder kann folgende Schritte aufweisen: eine Bestimmung einer auf ein Spannelement wirkenden Kraft bzw. von auf Spannelemente wirkenden Kräften; eine Bestimmung einer dem Spannelement zugeordneten Wegdifferenz bzw. von den Spannelementen zugeordneten Wegdifferenzen und/oder einer dem Spannelement zugeordneten Auslenkung bzw. von Spannelementen zugeordneten Auslenkungen aus einer Referenzposition; und gegebenenfalls eine Bestimmung einer Achslage einer Vorrichtung zum Spannen von Bändern, wobei wenigstens eine der vorgenannten Bestimmungen berührungslos erfolgt. Die Vorteile des offenbarten Verfahrens ergeben sich analog zu denen der Vorrichtung zum Spannen von Bändern bzw. der Einrichtung zur Erfassung einer Kraft-Weg-Kennlinie. Stellvertretend sei an dieser Stelle nur auf die günstigen Sterilitäts-Eigenschaften verwiesen.

Bevorzugt werden die Verfahrensschritte, die eine Bestimmung eines Meßwerts oder eine anderweitige Bestimmung aufweisen, in etwa gleichzeitig durchgeführt. Wie bereits vorstehend erwähnt, bedeutet in etwa gleichzeitig dabei lediglich durch Totzeiten bzw. Rechenzeiten der Recheneinheit, Signallaufzeiten, sowie durch eventuelle Totzeiten der Meßgeräte zeitlich getrennt. Der Vorteil ist die hohe Präzision der resultierenden Kurve.

Mindestens eine der Bestimmungen weist ein optisches und ggf. zuzätzlich ein magnetisches und/oder elektromagnetisches Meßverfahren auf. Auch an dieser Stelle sei bezüglich der Vorteile auf die im Rahmen der Beschreibung der Vorrichtung genannten Vorzüge verwiesen.

Die Bestimmung der vorstehend näher erläuterten Meßwerte wird vorzugsweise iterativ durchgeführt, wobei so eine Kraft-Weg-Kennlinie erfaßt wird, sobald die jeweilige auf das korrespondierende Spannelement wirkende Kraft variiert wird. Mittels einer vom Operateur bestimmbaren Punktdichte kann in Intervallen, die von besonderem Interesse sind, durch eine erhöhte Punktdichte bzw. erhöhte Dichte von Meßwerten die Präzision nochmals gesteigert werden, in Intervallen, die von geringerem Interesse sind, kann durch die Aufnahme einer geringeren Punktdichte die Operationsdauer verkürzt werden.

Bei Anliegen einer konstanten Kraft am Spannelement bzw. bei Anliegen von konstanten Kräften an den Spannelementen wird vorzugsweise die Auslenkung desselben bzw. derselben aus einer Referenzposition, beispielsweise einer Null-Position, oder aber im Falle einer konstanten Auslenkung aus einer Referenz- bzw. Null-Position die anliegende Kraft bzw. die anliegenden Kräfte in Abhängigkeit der Zeit erfaßt. Damit lassen sich Aufschlüsse über sich die viskoelastischen Eigenschaften des biologischen Gewebes gewinnen.

Weitere Vorteile und Zweckmäßigkeiten der Erfindung ergeben sich aus den abhängigen Ansprüchen sowie der nachfolgenden Beschreibung bevorzugter Ausführungsformen anhand der beigefügten Figuren. Diese zeigen in:
- Fig. 1: eine erste Ausführungsform einer erfindungsgemäßen Vorrichtung zum Spannen von Bändern;
- Fig. 2: eine zweite Ausführungsform einer erfindungsgemäßen Vorrichtung zum Spannen von Bändern;
- Fig. 3a, b: eine Ausführungsform einer Einrichtung zu Erfassen eines Kraft-Weg-Verhältnisses oder einer Kraft-Weg-Kennlinie, wobei diese Einrichtung eine Vorrichtung zum Spannen von Bändern gemäß Fig. 1 umfaßt; und
- Fig. 4: eine beispielhafte Kraft-Weg-Kennlinie eines Kapsel- und Band-Apparates.

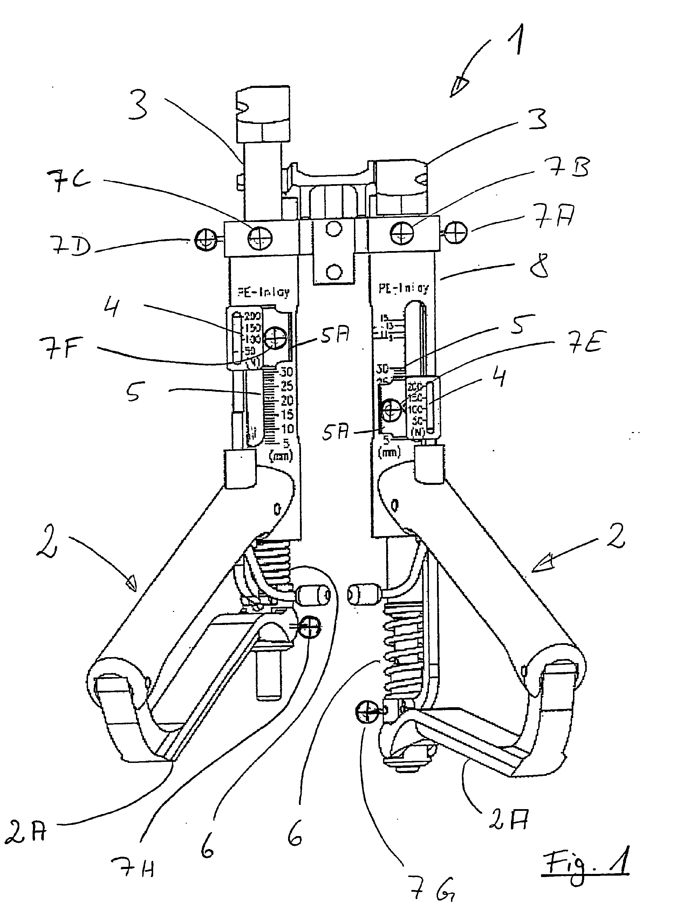

Die in Fig. 1 dargestellte Vorrichtung 1 zum Spannen von Bändern, genauer gesagt zum Spannen von Gelenkbändern und zum Spannen eines Kapsel-Apparates eines Gelenkes, weist zwei Handhaben 2 auf. Jede dieser Handhaben 2 steht mit einem jeweils zugeordneten Spannelement 3 in Wirkeingriff. Jeder der beiden Handhaben 2 sind ferner jeweils eine Kraftanzeige 4 und eine Weganzeige 5 zugeordnet. Sowohl die Kraftanzeigen 4 als auch die Weganzeigen 5 weisen jeweils eine zugeordnete Skala zum Ablesen der Werte auf. Die Kraftanzeigen 4 stellen jeweils die mittels der zugeordneten Handhabe 2, genauer gesagt die mittels eines im wesentlichen vertikal bewegbaren Teils 2A der Handhabe 2 auf das jeweils zugeordnete Spannelement 3 aufgebrachte Kraft dar. Die Weganzeigen 5 repräsentieren die Auslenkung des jeweils zugeordneten Spannelements 3, die durch die auf der jeweiligen Kraftanzeige 4 angezeigte Kraft bewirkt wird.

Wie vorstehend erläutert, ist das Spannelement 3 im vorliegenden Ausführungsbeispiel mechanisch über die Handhaben 2 betätigbar. Es sei angemerkt, daß auch eine elektromotorische, hydraulische, pneumatische oder hydro-pneumatische Betätigung denkbar wäre. Eine Betätigungseinrichtung für das jeweils zugeordnete Spannelement 3 umfaßt ein krafterzeugendes Element, das im vorliegenden Ausführungsbeispiel in Form einer Schraubendruckfeder 6 vorliegt. Wird die Schraubendruckfeder mittels der Handhabe 2, genauer gesagt mittels des bewegbaren Teils 2A der Handhabe 2 zusammengedrückt (vgl. hierzu insbesondere die linke Seite in Fig. 1), so wirkt auf das jeweils zugeordnete Spannelement 3 eine Kraft. Aufgrund der anliegenden Kraft wird das Spannelement 3 aus seiner Referenzposition in Form einer Null-Position bzw. Null-Lage ausgelenkt.

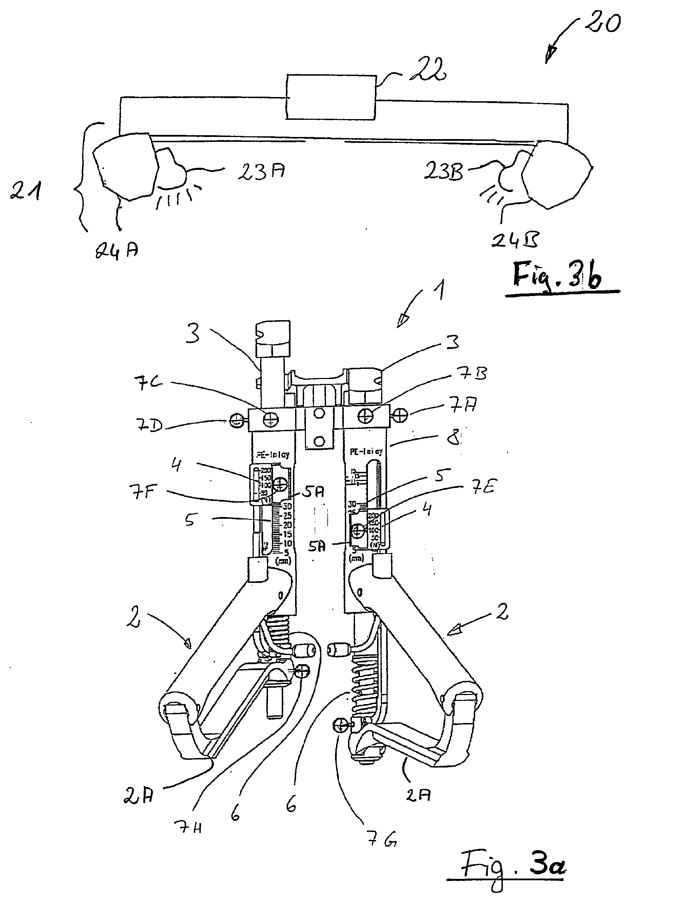

Weiterhin sind an der Vorrichtung 1 acht Geber 7A-7H angebracht, welche durch ein der Vorrichtung zugeordnetes Meßwert-Erfassungssystem (vgl. hierzu auch Fig. 3a), das nachstehend näher erläutert wird, beriihrungslos erfaßbar ist. Im vorliegenden Ausführungsbeispiel sind die Geber 7A-7D an einem Gehäuse der Vorrichtung 1 angebracht und dienen der Bestimmung der Achsausrichtung der Vorrichtung 1. Zwei weitere Geber 7E und 7F sind jeweils auf einem der Weganzeige 5 zugeordneten Schlitten 5A angebracht und können zur Erfassung der Auslenkung des jeweiligen Spannelements 3 aus seiner Referenzposition erfaßt werden. Zwei weitere Geber 7G und 7H sind am unteren beweglichen Teil der Handhabe 2 angebracht und dienen der Feststellung der Federspannung mittels einer Feststellung der Länge der Feder. Mittels der bekannten Federkonstante bzw. Federcharakteristik der jeweiligen Schraubendruckfeder 6 und den mittels der Geber 7G und 7H ermittelten Längen der Feder läßt sich die Kraft, die auf das jeweilige Spannelement 3 ausgeübt wird, einfach ermitteln. Die Geber 7A-7H weisen zur (optischen) Erfassung mittels der zugehörigen Einrichtung 20 (vgl. Fig. 3b) passive optische Elemente in Form von retro-reflektierenden Kugeln auf.

Es sei noch kurz erwähnt, daß die Geber 7A-7H teilweise oder auch gesamt selbstverständlich auch magnetische und/oder elektromagnetische Elemente aufweisen können, womit eine ebenso präzise Detektion der Positionen möglich ist. Ferner sei auch darauf hingewiesen, daß selbstverständlich nicht alle Geber 7A-7H nach demselben Wirkprinzip, d.h. also optisch, magnetisch und/oder elektromagnetisch funktionieren müssen, jedoch ist es vorzuziehen, daß sämtliche Geber demselben Wirkprinzip unterliegen. Dies stellt eine Positionsbestimmung mit minimalem apparativem Aufwand sicher.

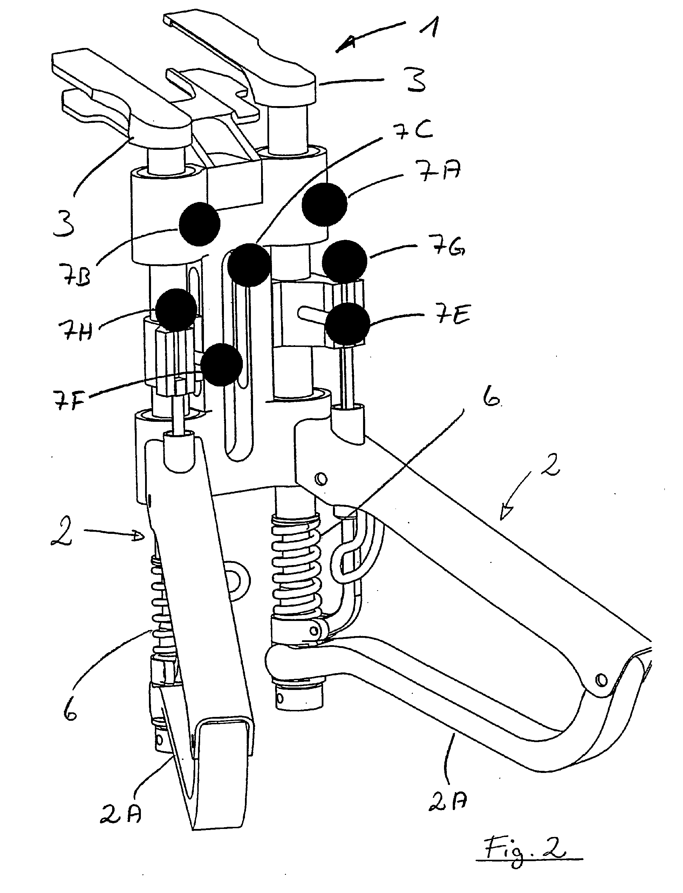

Die in Fig. 2 dargestellte Vorrichtung 1 zum Spannen von Bändern stellt eine weitere mögliche Ausführungsform einer erfindungsgemäßen Vorrichtung 1 zum Spannen von Bändern dar. Sie ist im Vergleich zu der in Fig. 1 dargestellten Vorrichtung 1 etwas einfacher aufgebaut. Im wesentlichen umfaßt die Vorrichtung 1 gemäß der zweiten Ausführungsform wiederum zwei Handhaben 2 mit einem jeweils zugeordneten bewegbaren Teil 2A sowie jeweils eine zugeordnete Schraubendruckfeder 6, welche auf ein zugeordnetes Spannelement 3 eine Kraft ausübt. Das Wirkungsprinzip entspricht dem der Vorrichtung 1 gemäß Fig. 1. Zur Bestimmung der Achsausrichtung sind drei Geber 7A bis 7C an einem Gehäuse der Vorrichtung 1 angebracht. Zwei weitere Geber 7E und 7F dienen der Erfassung der Auslenkung des jeweiligen Spannelements 3 aus seiner Referenzposition. Schließlich sind nochmals zwei weitere Geber 7G und 7H an der Vorrichtung 1 angebracht, die analog zur in Fig. 1 dargestellten Vorrichtung der Feststellung der Federspannung mittels einer Feststellung der Länge der Feder dienen. Wie vorstehend erwähnt, weist die Ausführungsform gemäß Fig. 2 einen etwas einfacheren Aufbau im Vergleich zu der Ausführungsform gemäß Fig. 1 auf. Eine jeweilige der Kraftanzeige und der Weganzeige zugeordnete Skala entfällt, wodurch ein konstruktiv einfacherer Aufbau sowie ein Aufbau mit einem geringeren Gesamtgewicht gewährleistet ist. Eine Vorrichtung 1 gemäß Fig. 2 ist vorzugsweise in Verbindung mit einer Einrichtung 20 (wird in der Folge näher erläutert) zum Erfassen einer Kraft-Weg-Kennlinie verwendbar, während die Ausführungsform gemäß Fig. 1 auch die Möglichkeit einer visuellen Ablesung durch den Operateur im Falle eines Ausfalls der Einrichtung 20 oder in dem Fall, daß keine Einrichtung 20 zur Verfügung steht, bildet.

Eine vollständige Einrichtung zum Erfassen eines Kraft-Weg-Verhältnisses oder einer Kraft-Weg-Kennlinie eines oder mehrerer Bänder, insbesondere Gelenkbänder, die, wie bereits vorstehend erwähnt, tierischer oder menschlicher Natur sein können, ist die in Fig. 3b dargestellte Einrichtung 20. Diese umfaßt in der vorliegenden Ausführungsform die bereits vorstehend beschriebene Vorrichtung 1 zum Spannen von Bändern, sowie Mittel zur Erfassung der Geber in Form einer Stereo-Kamera-Anordnung 21 sowie Mittel zur Auswertung der von der Stereo-Kamera-Anordnung 21 erfaßten Daten in Form einer Auswertungseinheit 22. Die wesentlichen Komponenten der Stereo-Kamera-Anordnung 21 sind zwei IR-Beleuchtungsquellen 23A und 23B, sowie zwei IR-Kameras 24A und 24B. Selbstverständlich ist auch die Verwendung einer anderen Vorrichtung 1 zum Spannen von Bändern denkbar, beispielsweise die Verwendung einer Vorrichtung 1 gemäß Fig. 2.

Die Auswertungseinheit 22 umfaßt eine Recheneinheit, in der ein spezielles Auswertungsprogramm implementiert ist, welches eine zusammenfassende Auswertung der von den Gebern 7A-7H kommenden Licht- bzw. IR-Signale zur Erstellung eines Positionsdatensatzes sowohl hinsichtlich der Achsausrichtung der Vorrichtung 1, sowie auch der jeweiligen auf die Spannelemente 3 ausgeübten Kraft, sowie deren Auslenkung aus der Referenzposition erlaubt.

Ferner umfaßt die Auswertungseinheit 22 einen Speicher zur Speicherung der erfaßten und auch der ausgewerteten Daten. Gespeichert werden ferner Referenzwerte und spezifische Daten, insbesondere Referenzdaten hinsichtlich der Raumlage des zu vermessenden Band-Apparates.

Eine Messung der drei vorgenannten Größen, nämlich der auf die Spannelemente 3 jeweils wirkenden Kräfte, der von den jeweiligen Spannelementen 3 zurückgelegten Wegstrecke bzw. der Auslenkung der jeweiligen Spannelemente 3 aus der Referenzposition, sowie eine Messung der Achsausrichtung der Vorrichtung 1 zum Spannen von Bändern, erfolgt in etwa zeitgleich. Zeitgleich bedeutet in diesem Sinne, daß lediglich Totzeiten der jeweiligen Meßapparaturen, Signallaufzeiten, sowie bei einer sequentiellen Abarbeitung des Auswertungsprogramms die vom Prozessor der Auswertungseinheit 22 benötigte Zeit, zwischen den einzelnen Messungen liegt. Bei den üblichen Taktfrequenzen von Prozessoren und den geringen Totzeiten von modernen Meß-Apparaturen können die Messungen in guter Näherung als zeitgleich angesehen werden.

Die Einrichtung 20 ist ferner dazu ausgelegt, zeitabhängige Änderungen der auf die jeweiligen Spannelemente 3 ausgeübten Kräfte und/oder Auslenkungen der krafterzeugenden Elemente aus ihrer Referenzposition in Abhängigkeit der Zeit zu messen. Dies heißt also, daß beispielsweise bei konstant gehaltener Kraft auf ein Spannelement 3 die Auslenkung aus dessen Referenzposition in Abhängigkeit von der Zeit aufgezeichnet wird bzw. bei einer konstant gehaltenen Auslenkung aus einer Referenzposition die auf das jeweilige Spannelement 3 wirkende Kraft in Abhängigkeit der Zeit aufgezeichnet wird. Damit lassen sich zeitabhängige Veränderungen im Band- und Kapsel-Apparat eines Patienten sichtbar machen und somit viskoelastische Eigenschaften des biologischen Gewebes messen und beurteilen.

Es sei angemerkt, daß grundsätzlich mit einer einzigen Signalisierung drei Raumskalen abgelesen werden können. In einer anderen Ausführungsform der hier vorgestellten Vorrichtung 1 zum Spannen von Bändern bzw. der Einrichtung 20 zum Erfassen eines Kraft-Weg-Verhältnisses oder einer Kraft-Weg-Kennlinie werden Kraft- und Wegmessung mit einer einzigen Signalisierung bestimmt. Hierzu ist die Wegmeßskala orthogonal zur Kraftmeßskala orientiert. Gleichzeitig kann bei Bedarf die Ausrichtung der Anatomie aufgezeichnet werden (Beinachse in x° Flexion, d.h. Beugewinkel, Extension).

Ferner sei angemerkt, daß bei den vorstehend erwähnten Ausführungsformen (wie bereits beschrieben erfolgt die Bestimmung der Kraft auf "indirektem" Wege über die Bestimmung einer Strecke, nämlich der Federlänge) die Genauigkeit der Kraftmessung aufgrund einer Untersetzung der beweglichen Teile hoch ist. Die Untersetzung wirkt derart, daß ein Millimeter Skalenänderung in den vorstehend erwähnten Ausführungsbeispielen einer Änderung in der Federlänge von 0,1 mm entspricht. Dies verringert Fehler in der Kraftmessung, insbesondere durch Meßtoleranzen bedingte Fehler. Bei der Kraftmessung wird, wie bereits erwähnt, mit der Kennlinie der Federspannung der Weg in eine Kraft umgerechnet, wobei es sich praktisch um eine indirekte optische Ablesung einer Kraft handelt.

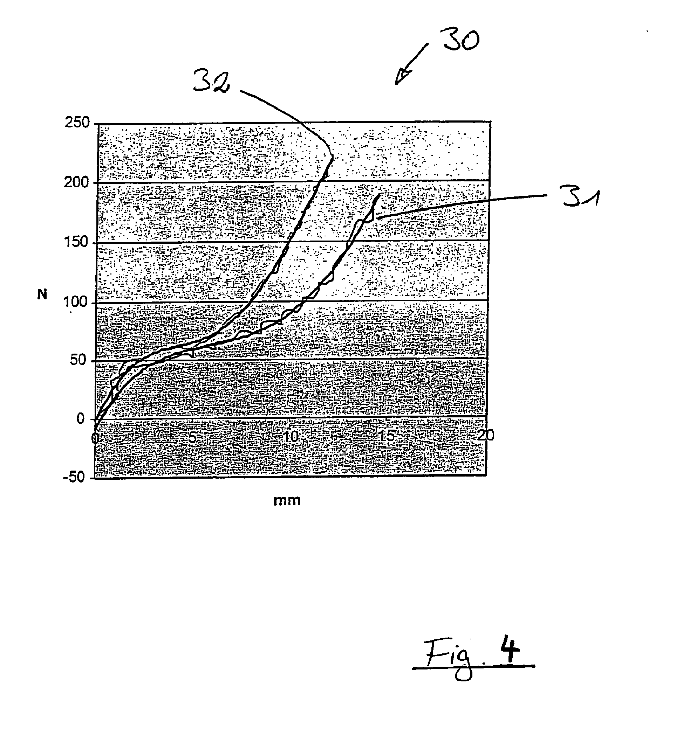

Zum Abschluß sei auf das Verfahren, auf dem die vorstehend beschriebene Vorrichtung 1 bzw. Einrichtung 20 zur Erfassung eines Kraft-Weg-Verhältnisses oder einer Kraft-Weg-Kennlinie basiert, eingegangen. Beispielhaft sei auf ein Diagramm 30 verwiesen, welches in Fig. 4 zwei Kraft-Weg-Kennlinien 31 und 32 darstellt. Im vorliegenden Beispiel handelt es sich um Kraft-Weg-Kennlinien 31, 32 eines menschlichen Knies, wobei die Kraft-Weg-Kennlinie 31 der lateralen Seite des Knies, sowie die Kraft-Weg-Kennlinie 32 der medialen Seite des Knies entspricht. Die "gezackten" Kurven der Kraft-Weg-Kennlinien 31 und 32 entsprechen den Meßkurven, die glatten, durchgezogenen Linien entsprechen jeweils einem polynomischen Fit. Aus einer derartigen Kraft-Weg-Kennlinie 31, 32 kann der Operateur die ideale Vorspannung für den Band-Kapsel-Apparat entnehmen.

Das Verfahren zur Erfassung eines Kraft-Weg-Verhältnisses bzw. einer Kraft-Weg-Kennlinie 31, 32 umfaßt im wesentlichen die folgenden Schritte: eine Bestimmung der jeweiligen auf ein Spannelement 3 wirkenden Kraft, eine Bestimmung der dem jeweiligen Spannelement 3 zugeordneten Auslenkung aus einer Referenzposition und eine Bestimmung einer Achslage der Vorrichtung 1 zum Spannen von Bändern. Die Bestimmung der jeweiligen Größen erfolgt berührungslos. Die vorstehend näher erläuterten Bestimmungen der Größen erfolgen auch in etwa gleichzeitig, wobei, wie bereits vorstehend erwähnt, gleichzeitig in dem Sinne aufzufassen ist, daß die einzelnen Bestimmungen lediglich durch eventuelle Totzeiten der Meßapparaturen, Signallaufzeiten, sowie durch die Auswertungseinheit bedingte Zeitdifferenzen getrennt sind. Die durch die Auswertungseinheit 22 bedingten Zeitdifferenzen sind im wesentlichen dadurch bestimmt, daß die Befehle zur Meßwertbestimmung bzw. -verarbeitung teilweise sequentiell in der Auswertungseinheit 22 abgearbeitet werden. Durch die iterative Durchführung der vorstehend näher erwähnten Schritte gelangt man zu den in Fig. 4 dargestellten Kraft-Weg-Kennlinien 31 und 32. Alternativ oder zusätzlich, je nach Bedarf des Operateurs, kann bei Anliegen einer konstanten Kraft am Spannelement 3 die Auslenkung desselben aus seiner Referenzposition oder bei einer konstanten Auslenkung aus der vorgenannten Referenzposition die am Spannelement 3 anliegende Kraft in Abhängigkeit der Zeit erfaßt werden, was, wie bereits vorstehend erwähnt, der Messung und Beurteilung von viskoelastischen Eigenschaften des biologischen Gewebes dient.

### Bezugszeichenliste

- 1: Vorrichtung zum Spannen von Bändern
- 2: Handhabe
- 2A: bewegbarer Teil der Handhabe 2
- 3: Spannelement
- 4: Kraftanzeige
- 5: Weganzeige
- 5A: der Weganzeige 5 zugeordneter Schlitten
- 6: Schraubendruckfeder
- 7A-7H: Geber
- 8: Gehäuse
- 20: Einrichtung zum Erfassen einer Kraft-Weg-Kennlinie
- 21: Stereo-Kamera-Anordnung
- 22: Auswertungseinheit
- 23A, 23B: IR-Beleuchtungsquelle
- 24A, 24B: IR-Kamera
- 30: Diagramm
- 31, 32: Kraft-Weg-Kennlinie

## Patentansprüche

1. Vorrichtung (1) zum Spannen von Bändern, insbesondere Gelenkbändern, eines menschlichen oder eines tierischen Körpers mit mindestens einer Handhabe (2), wobei die bzw. jede Handhabe (2) mit jeweils einem dieser zugeordneten Spannelement (3) in Wirkeingriff steht, und wobei der bzw. jeder Handhabe (2) jeweils eine Kraftanzeige (4), welche eine bei einem Spannen eines oder mehrerer Bänder auf ein bzw. jedes der bzw. jeder Handhabe (2) zugeordnetes Spannelement (3) ausgeübte Kraft anzeigt, und eine Weganzeige (5), die die Auslenkung des jeweiligen Spannelements (3) aus einer Referenzposition anzeigt, wobei der bzw. jeder Kraftanzeige (4) und der bzw. jeder Weganzeige (5) jeweils mindestens ein Geber (7A-7H) zugeordnet ist bzw. sind, welche durch ein der Vorrichtung (1) zugeordnetes Messwerterfassungssystem (20) berührungslos erfassbar sind,
**dadurch gekennzeichnet, das**
wenigstens ein Teil der vorhandenen Geber (7A-7H) retro-reflektierende Kugeln aufweist.

2. Vorrichtung (1) nach Anspruch 1,
**dadurch gekennzeichnet, dass**
der/die Geber (7A-7H) magnetische und/oder elektromagnetische Elemente aufweisen.

3. Vorrichtung (1) nach Anspruch 1 oder 2,
**dadurch gekennzeichnet, dass**
das bzw. jedes Spannelement (3) mechanisch, elektromotorisch, hydraulisch, pneumatisch oder hydro-pneumatisch betätigbar ist.

4. Vorrichtung (1) nach einem der vorangehenden Ansprüche,
**dadurch gekennzeichnet, dass**
eine Betätigungseinrichtung für das Spannelement (3) ein krafterzeugendes Element umfasst, wobei die auf das jeweilige Spannelement (3) wirkende Kraft durch eine Messung einer dem jeweiligen krafterzeugenden Element zugeordneten Wegdifferenz und/oder Auslenkung aus einer Referenzposition bestimmbar ist.

5. Vorrichtung (1) nach Anspruch 4,
**dadurch gekennzeichnet, dass**
das krafterzeugende Element eine Schraubendruckfeder (6) ist.

6. Vorrichtung (1) nach einem der vorangehenden Ansprüche,
**gekennzeichnet durch**
mindestens zwei weitere Geber (7A-7D) vorgesehen sind, die zur Bestimmung einer Achsausrichtung der Vorrichtung (1) dienen.

7. Vorrichtung (1) nach Anspruch 6,
**dadurch gekennzeichnet, dass**
jeder der weiteren Geber (7A-7D) für die Bestimmung der Achsausrichtung magnetische und/oder elektromagnetische und/oder optische Elemente aufweist.

8. Einrichtung (20) zum Erfassen eines Kraft-Weg-Verhältnisses oder einer Kraft-Weg-Kennlinie eines oder mehrerer tierischer oder menschlicher Bänder, insbesondere Gelenkbänder,
**gekennzeichnet durch**
- eine Vorrichtung (1) zum Spannen von Bändern nach einem oder mehreren der Ansprüche 1 bis 7;
- Mittel (21) zur Erfassung der Geber; und
- Mittel (22) zur Auswertung der erfassten Daten.

9. Einrichtung (20) nach Anspruch 8,
**dadurch gekennzeichnet, dass**
sie ferner einen Speicher zur Speicherung der erfassten und/oder ausgewerteten Daten und/oder zur Speicherung von Referenzwerten und/oder spezifischer Daten hinsichtlich der Raumlage des zu vermessenden Band-Apparates aufweist.

10. Einrichtung (20) nach Anspruch 8 oder 9,
**dadurch gekennzeichnet, dass**
eine Messung der auf ein Spannelement (3) wirkenden Kraft bzw. der auf Spannelemente (3) wirkenden Kräfte,
eine Messung einer dem Spannelement (3) zugeordneten Wegdifferenz und/oder Auslenkung aus einer Referenzposition bzw. Spannelementen (3) zugeordnete Wegdifferenzen und/oder Auslenkungen, sowie
eine Messung der Achsausrichtung der Vorrichtung (1) zum Spannen von Bändern in etwa zeitgleich erfolgen.

11. Einrichtung (20) nach einem der Ansprüche 8 bis 10,
**gekennzeichnet durch**
Mittel zur Erfassung zeitabhängiger Änderungen von auf zugeordnete Spannelemente (3) ausgeübten Kräften und/oder krafterzeugenden Elementen zugeordneten Wegdifferenzen und/oder Auslenkungen aus einer Referenzposition.

12. Einrichtung (20) nach einem der Ansprüche 8 bis 11,
**dadurch gekennzeichnet, dass**
sie zur Erfassung von optischen Gebern eine Stereo-Kamera (24A, 24B) umfasst.

## Claims

1. Appliance (1) for tensioning ligaments, especially articular ligaments, of a human or animal body, having at least one handle (2), the or each handle (2) being in operative engagement with, in each case, a tensioning element (3) associated therewith, and with the or each handle (2) in each case a force indicator (4), which indicates a force exerted on a or each tensioning element (3) associated with the or each handle (2) on tensioning of one or more ligaments, and a displacement indicator (5), which indicates the excursion of the tensioning element (3) in question from a reference position, wherein
with the or each force indicator (4) and with the or each displacement indicator (5) there is/are associated in each case at least one marker (7A-7H), which is arranged to be detected without contact by a measurement value ascertainment system (20) associated with the appliance (1),
**characterised in that**
at least a sub-set of the markers (7A-7H) present comprises retroreflective balls.

2. Appliance (1) according to claim 1,
**characterised in that**
the marker(s) (7A-7H) comprise magnetic and/or electromagnetic elements.

3. Appliance (1) according to claim 1 or 2,
**characterised in that**
the or each tensioning element (3) is mechanically, electromotively, hydraulically, pneumatically or hydro-pneumatically actuatable.

4. Appliance (1) according to one of the preceding claims,
**characterised in that**
an actuation device for the tensioning element (3) comprises a force-producing element, the force acting on the tensioning element (3) in question being determinable by measurement of a displacement difference and/or excursion from a reference position, which displacement difference and/or excursion is associated with the force-producing element in question.

5. Appliance (1) according to claim 4,
**characterised in that**
the force-producing element is a helical compression spring (6).

6. Appliance (1) according to one of the preceding claims,
**characterised in that**
at least two further markers (7A-7D) are provided, which serve for determining axis orientation of the appliance (1).

7. Appliance (1) according to claim 6,
**characterised in that**
each of the further markers (7A-7D) for determining axis orientation comprises magnetic and/or electromagnetic and/or optical elements.

8. Device (20) for ascertaining a force-displacement relationship or a force-displacement characteristic curve of one or more animal or human ligaments, especially articular ligaments,
**characterised by**
- an appliance (1) for tensioning ligaments, in accordance with one or more of claims 1 to 7;
- means (21) for detecting the markers; and
- means (22) for evaluating the data ascertained.

9. Device (20) according to claim 8,
**characterised in that**
it further comprises a memory for storing the ascertained and/or evaluated data and/or for storing reference data and/or specific data relating to the spatial location of the ligament apparatus being surveyed.

10. Device (20) according to claim 8 or 9,
**characterised in that**
a measurement of the force acting on a tensioning element (3) or of the forces acting on tensioning elements (3),
a measurement of a displacement difference and/or excursion from a reference position, associated with the tensioning element (3), or of displacement differences and/or excursions associated with tensioning elements (3), and also a measurement of the axis orientation of the appliance (1) for tensioning ligaments are carried out approximately simultaneously.

11. Device (20) according to one of claims 8 to 10,
**characterised by**
means for ascertaining time-dependent changes in forces exerted on associated tensioning elements (3) and/or in displacement differences and/or excursions from a reference position, which displacement differences and/or excursions are associated with force-producing elements.

12. Device (20) according to one of claims 8 to 11,
**characterised in that**
it includes a stereo camera (24A, 24B) for detecting optical markers.

## Revendications

1. Dispositif (1) pour tendre des ligaments, en particulier des ligaments d'articulation d'un corps humain ou d'un corps animal avec au moins une manette (2), dans lequel la ou chaque manette (2) se trouve en prise opérationnelle avec respectivement un élément de tension (3) qui lui est affecté, et dans lequel la ou chaque manette (2) un affichage dynamométrique (4), lequel affiche une force exercée lors d'une tension d'un ou plusieurs ligaments sur un ou chaque élément de tension (3) affecté à la ou chaque manette (2), et un affichage de course (5), qui affiche l'élongation de l'élément de tension (3) respectif depuis une position de référence, dans lequel au moins un capteur (7A-7H) est ou sont respectivement affecté(s) à le ou chaque affichage dynamométrique (4) et à le ou chaque affichage de course (5), lesquels peuvent être saisis sans contact par un système de saisie des valeurs de mesures (20) affecté au dispositif (1),
**caractérisé en ce que**
au moins une partie des capteurs (7A-7H) existants présentent des billes rétroréflectrices.

2. Dispositif (1) selon la revendication 1,
**caractérisé en ce que**
le/les capteurs (7A-7H) présentent des éléments magnétiques et/ou électromagnétiques.

3. Dispositif (1) selon la revendication 1 ou 2,
**caractérisé en ce que**
le ou chaque élément de tension (3) peut être actionné de manières mécanique, électromotrice, hydraulique, pneumatique ou hydropneumatique.

4. Dispositif (1) selon l'une quelconque des revendications précédentes,
**caractérisé en ce que**
un équipement d'actionnement pour l'élément de tension (3) comprend un élément générateur de force, dans lequel la force agissant sur l'élément de tension (3) respectif peut être déterminée par la mesure d'une différence de course et/ou d'une élongation affectée à l'élément générateur de force respectif depuis une position de référence.

5. Dispositif (1) selon la revendication 4,
**caractérisé en ce que**
l'élément générateur de force est un ressort hélicoïdal de compression (6).

6. Dispositif (1) selon l'une quelconque des revendications précédentes,
**caractérisé en ce que**
au moins deux autres capteurs (7A-7D) sont prévus, qui servent à la détermination d'une orientation des axes du dispositif (1).

7. Dispositif (1) selon la revendication 6,
**caractérisé en ce que**
chacun des autres capteurs (7A-7D) présente des éléments magnétiques et/ou électromagnétiques et/ou optiques pour la détermination de l'orientation des axes.

8. Equipement (20) destiné à saisir un rapport force/course ou une courbe caractéristique force-course d'un ou plusieurs ligaments animaux ou humains, en particulier de ligaments d'articulation,
**caractérisé par**
- un dispositif (1) pour tendre des ligaments selon une ou plusieurs des revendications 1 à7;
- des moyens (21) pour la saisie des capteurs ; et
- de moyens (22) pour l'évaluation des données saisies.

9. Equipement (20) selon la revendication 8,
**caractérisé en ce que**
il présente en outre une mémoire pour l'enregistrement des données saisies et/ou évaluées et/ou pour l'enregistrement de valeurs de référence et/ou de données spécifiques concernant la position dans l'espace de l'appareil ligamentaire à mesurer.

10. Equipement (20) selon la revendication 8 ou 9,
**caractérisé en ce que**
une mesure de la force agissant sur un élément de tension (3) ou des forces agissant sur des éléments de tension (3),
une mesure d'une différence de course affectée à l'élément de tension (3) et/ou d'une élongation depuis une position de référence ou de différences de course affectées à des éléments de tension (3) et/ou d'élongations, ainsi que
une mesure de l'orientation des axes du dispositif (1) pour tendre des ligaments s'effectuent sensiblement simultanément.

11. Equipement (20) selon l'une quelconque des revendications 8 à 10,
**caractérisé par**
des moyens pour la saisie de modifications en fonction du temps de forces exercées sur des élément de tension (3) affectés et/ou de différences de courses affectées à des éléments générateurs de force et/ou d'élongations depuis une position de référence.

12. Equipement (20) selon l'une quelconque des revendications 8 à 11,
**caractérisé en ce que**
il comprend une caméra stéréoscopique (24A, 24B) pour la saisie de capteurs optiques.
